# FASCICULE DE BREVET EUROPEEN

(11) **EP 0 976 393 B1**
(45) Date de publication et mention de la délivrance du brevet: **02.01.2003**
(21) Numéro de dépôt: 99401698.8
(22) Date de dépôt: 07.07.1999
(51) Int. Cl.: A61K 7/50, A61K 7/06

(54) **Composition contenant un agent opacifiant ou nacrant et au moins un alcool gras**
Zusammensetzung die ein Trübungs- oder Perlglanzmittel und mindestens einen Fettalkohol enthällt
Composition containing an opacifying or pearlescent agent and at least one fatty alcohol

(30) Priorité: 27.07.1998 FR 9809562
(43) Date de publication de la demande: 02.02.2000
(73) Titulaire: L'OREAL, 75008 Paris (FR)
(72) Inventeur: Müller, Rainer, 76344 Leopoldshafen (DE); Beauquey, Bernard, 92110 Clichy (FR)
(74) Mandataire: Le Blainvaux Bellegarde, Françoise

(56) Documents cités:
- EP-A- 0 457 688
- EP-A- 0 498 716
- WO-A-98/03155
- DE-A- 19 622 968
- DATABASE WPI Week 8901 Derwent Publications Ltd., London, GB; AN 89-004428 XP002104841 "Hair treatment agent - comprising alkyl quaternary ammonium salt, polypeptide, stearyl alcohol and behenyl alcohol" & JP 63 284113 A (KANEBO), 21 novembre 1988 (1988-11-21)
- DATABASE WPI Week 9007 Derwent Publications Ltd., London, GB; AN 90-049552 XP002104839 "New detergent compsn. for skin - contg. higher alcohol e.g. behenyl alcohol, unstd. fatty acid salt and surfactant e.g. poly-oxy-ethylene alkyl ether, etc." & JP 02 004709 A (TOYO BEAUTY), 9 janvier 1990 (1990-01-09)
- DATABASE WPI Week 8515 Derwent Publications Ltd., London, GB; AN 85-089606 XP002104840 "New pearly agent compsn. for use in cosmetics - comprises mainly higher alcohol and nonionic surfactant" & JP 60 038310 A (KOBAYASHI KOSE), 27 février 1985 (1985-02-27)
- PATENT ABSTRACTS OF JAPAN vol. 1999, no. 2, 26 février 1999 (1999-02-26) & JP 10 298031 A (KAO), 10 novembre 1998 (1998-11-10)

## Description

La présente invention a trait à une composition comprenant au moins une base tensioactive, au moins un alcool gras ou un mélange d'alcools gras linéaires et saturés à longue chaîne comportant au moins 50% en poids d'un alcool ayant 22 atomes de carbone et au moins un agent opacifiant et/ou nacrant, à son utilisation en tant qu'agent de nacrage, à une composition cosmétique comprenant dans un milieu cosmétiquement acceptable au moins une base tensioactive, au moins un alcool gras ou un mélange d'alcools gras linéaires et saturés à longue chaîne comportant au moins 50% en poids d'un alcool ayant 22 atomes de carbone, au moins un agent opacifiant et/ou nacrant et au moins un agent de conditionnement des matières kératiniques. L'invention a également pour objet l'utilisation de ladite composition en tant qu'agent de suspension des agents de conditionnement insolubles.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents de conditionnement, notamment insolubles, pour faciliter le démêlage des cheveux et pour leur communiquer douceur, brillance et souplesse.

Compte tenu du caractère insoluble de certains agents de conditionnement tels que par exemple les silicones ou les huiles, on cherche à maintenir les agents de conditionnement en dispersion régulière dans le milieu sans cependant faire chuter la viscosité et les propriétés détergentes et moussantes des compositions. Les silicones doivent également être véhiculées sur les matières kératiniques traitées en vue de leur conférer, suivant l'application, des propriétés de douceur, de brillance et de démêlage.

On sait également que les produits en particulier cosmétiques ayant un aspect ou un effet irisé, moiré ou métallisé, sont largement appréciés par les consommateurs pour leur aspect esthétique et donnant une apparence de richesse au produit. Les agents qui apportent cet effet sont des agent de nacrage comprenant généralement des cristaux qui restent dispersés dans les compositions et qui réfléchissent la lumière.

Par le terme "agent de nacrage", on entend un agent produisant un aspect ou un effet irisé, moiré ou métallisé.

Il existe à ce jour peu de moyens pour maintenir efficacement en suspension les agents de conditionnement insolubles, car il s'agit là d'un problème difficile à résoudre ; à cet effet, on a déjà proposé l'utilisation des dérivés d'esters à longue chaîne ou des polysaccharides tels que la gomme de xanthane. Cependant, les dérivés d'esters à longue chaîne peuvent présenter des problèmes de cristallisation qui entraînent une évolution de la viscosité des compositions au cours du temps ; les agents gélifiants présentent également des inconvénients, à savoir, d'une part que la mousse des compositions détergentes contenant de la gomme de xanthane se développe difficilement (mauvais démarrage de mousse) et que, d'autre part, les compositions n'ont pas une texture lisse et s'écoulent par paquets, ce qui est peu apprécié des utilisateurs.

On connaît également les dérivés d'éthers ou de thioéthers à longue chaîne tels que ceux décrits dans les demandes EP457688 et WO98/03155. Cependant, ces derniers agents opacifient les compositions sans apporter de nacrage aux compositions ou pas suffisamment.

On a déjà essayé d'améliorer le nacrage en ajoutant des agents épaississants et/ou d'autres agents nacrants, mais ainsi la viscosité devient trop importante et/ou la composition n'est plus stable.

La demanderesse a découvert, ce qui fait l'objet de l'invention, que l'utilisation d'un alcool gras ou un mélange d'alcools gras linéaires et saturés à longue chaîne comportant au moins 50% en poids d'un alcool ayant 22 atomes de carbone permettait de nacrer et/ou améliorer le nacrage des compositions comprenant au moins une base tensioactive et au moins un agent opacifiant et/ou nacrant.

L'invention a donc pour objet des compositions comprenant au moins une base tensioactive, au moins un alcool gras ou un mélange d'alcools gras linéaires et saturés à longue chaîne comportant au moins 50% en poids d'un alcool ayant 22 atomes de carbone et au moins un agent opacifiant et/ou nacrant.

Les compositions selon l'invention peuvent être utilisées comme base nacrante des compositions cosmétiques pour procurer un effet nacré supérieur à celui obtenu par l'agent opacifiant et/ou nacrant.

L'invention a encore pour objet l'utilisation d'un alcool gras ou d'un mélange d'alcools gras linéaires et saturés à longue chaîne comportant au moins 50% en poids d'un alcool ayant 22 atomes de carbone et d'un agent opacifiant en tant que base nacrante.

Les compositions présentent une très bonne homogénéité et une bonne stabilité du nacrage, ainsi qu'une viscosité satisfaisante pour l'application sur les matières kératiniques.

D'autres objets de l'invention apparaîtront à la lecture de la description et des exemples qui suivent.

L'alcool gras ou le mélange d'alcools gras linéaires et saturés à longue chaîne comportant au moins 50% en poids d'un alcool ayant 22 atomes de carbone comporte plus particulièrement au moins 70% en poids d'alcool en C22 par rapport au poids total du mélange.
Généralement, le mélange d'alcools gras linéaires et saturés à longue chaîne contient des alcools gras en C16 à C24. Les alcools gras en C16 et C24 représentent chacun généralement moins de 2% en poids, les chaînes en C18 moins de 10% en poids du poids total du mélange.

De tels d'alcools gras sont notamment les produits commercialisés sous la dénomination NAFOL 1822 C par la société CONDEA qui contient environ 0,5% de C16, 4-6% de C18, 15-19% de C20, 74-78% de C22 et environ 1,5% de C24 ou le produit commercialisé sous la dénomination NAFOL 2298 par la société CONDEA qui contient 98% d'alcool en C22 ou sous la dénomination NAFOL 2298 qui contient 98% en poids d'alcool en C22.

Les agents nacrants et/ou opacifiant utilisables selon l'invention peuvent être choisis parmi :
A) les dialkyléthers gras solides à une température inférieure ou égale à environ 30°C tels que par exemple les dialkyléthers de formule (I) :

   R-O-R' (I)

   dans laquelle :
   R et R', identiques ou différents, désignent un radical alkyle, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ Plus particulièrement R et R' sont identiques.

   De façon préférentielle, R et R' désignent un radical stéaryle.
   Les dialkyléthers utilisables selon l'invention dans les compositions sont insolubles.
   Ces composés peuvent être préparés selon le procédé décrit dans la demande de brevet DE 41 27 230.
   Un distéaryléther utilisable dans le cadre de la présente invention, est notamment vendu sous la dénomination CUTINA KE 3178 par la société HENKEL.
B) les alcools ayant de 27 à 48 atomes de carbone et comportant un ou deux groupements éther et/ou thioéther ou sulfoxyde répondant à la formule (II) :

   R1-X-[C2H3(OH)]-CH2-Y-R2 (II)

   dans laquelle R1 et R2 désignent indépendamment l'un de l'autre, des groupements alkyles linéaires en C12 à C24 ;
   X désigne un atome d'oxygène, un atome de soufre, un groupement sulfoxyde ou méthylène ;
   Y désigne un atome d'oxygène, un atome de soufre, un groupement sulfoxyde ou méthylène ;
   dans le cas où Y désigne un groupement méthylène la somme du nombre d'atomes de carbone présents dans les groupements R1 et R2 a une valeur variant de 24 à 44 et de préférence de 28 à 40 inclus ;
   lorsque Y ne désigne pas un groupement méthylène, la somme des atomes de carbone présents dans les groupements R1 et R2 a une valeur variant de 24 à 44 et de préférence de 28 à 40 inclus ;
   lorsque X ou Y désigne sulfoxyde, Y ou X ne désigne pas soufre.

Les composés de formules (II) utilisés de préférence conformément à l'invention, sont ceux pour lesquels X désigne oxygène, Y désigne méthylène, et R1 et R2 désignent des radicaux ayant 12 à 22 atomes de carbone.
Ces composés peuvent être préparés selon le brevet EP 457 688.

Selon l'invention, l'alcool gras linéaire et saturé comportant au moins 50% en poids d'alcool contenant 22 atomes de carbone peut représenter de 0,5% à 15 % en poids, de préférence de 0,5 % à 5 % en poids, et encore plus préférentiellement de 0,5 % à 3 % en poids, du poids total de la composition finale.

Selon l'invention, l'agent nacrant et/ou opacifiant peut représenter de 0,5 % à 15% en poids, de préférence de 0,5 % à 5 % en poids, et encore plus préférentiellement de 1 % à 3 % en poids, du poids total de la composition finale.

Le rapport agent opacifiant/alcool gras en C22 est généralement compris entre 0,2 et 8 et de préférence entre 0,3 et 5.

Selon une variante préférée de l'invention, les compositions cosmétiques peuvent également contenir des agents de conditionnement des matières kératiniques.

L'invention a donc également pour objet de nouvelles compositions cosmétiques en particulier de lavage et de conditionnement moussantes comprenant dans un milieu cosmétiquement acceptable une base tensioactive, au moins un agents de conditionnement, au moins un alcool gras ou un mélange d'alcools gras linéaires et saturés à longue chaîne comportant au moins 50% en poids d'un alcool ayant 22 atomes de carbone et au moins un agent opacifiant et/ou nacrant.

Les compositions ainsi préparées possèdent en outre, de bonnes propriétés détergentes et moussantes et confèrent aux matières kératiniques, notamment aux cheveux et/ou à la peau, une grande douceur.

Ces compositions, lorsqu'elles sont appliquées sur les cheveux, possèdent, en plus de leurs propriétés lavantes, des propriétés de conditionnement des cheveux, c'est-à-dire que les cheveux traités sont lisses, se démêlent facilement, sont doux au toucher. Les cheveux ont un aspect naturel et non chargé.

Les compositions selon l'invention contenant des agents de conditionnement sont stables : en particulier, il ne se produit aucun relargage des agents de conditionnement ou d'épaississement incontrôlé de la composition au cours du temps. Les compositions présentent enfin une texture non filante et fondante. La mousse est aérée et se rince facilement.

Un autre objet de l'invention est constitué par le procédé de lavage et de conditionnement mettant en oeuvre de telles compositions.

L'invention a encore pour objet l'utilisation d'au moins un alcool gras ou un mélange d'alcools gras linéaires et saturés à longue chaîne comportant au moins 50% en poids d'un alcool ayant 22 atomes de carbone et d'au moins un agent opacifiant et/ou nacrant comme agent de mise en suspension d'un agent de conditionnement insoluble dans une composition cosmétique en particulier de lavage et de conditionnement moussante contenant, dans un milieu aqueux cosmétiquement acceptable, une base tensioactive.

Lorsque la composition contient au moins un agent de conditionnement, ils sont généralement choisis parmi les poly-α-oléfines, les huiles fluorées, les cires fluorées, les gommes fluorées, les esters d'acides carboxyliques, les polymères cationiques, les silicones, les huiles minérales, végétales ou animales, les céramides, les pseudocéramides et leurs mélanges.

Le milieu cosmétiquement acceptable est de préférence constitué par de l'eau ou un mélange d'eau et de solvants cosmétiquement ou dermatologiquement acceptables tels que des monoalcools, des polyalcools, des éthers de glycol ou des esters d'acides gras, qui peuvent être utilisés seuls ou en mélange.

On peut citer plus particulièrement les alcools inférieurs tels que l'éthanol, l'isopropanol, les polyalcools tels que le diéthylèneglycol, la glycérine, le sorbitol, les éthers de glycol, les alkyléthers de glycol ou de diéthylèneglycol.

La composition de l'invention peut également contenir au moins un additif choisi parmi les séquestrants, les adoucissants, les modificateurs de mousse, les colorants, d'autres agents nacrants, les agents hydratants, les agents antipelliculaires ou anti-séborrhéiques, d'autres agents de mise en suspension, les acides gras à chaînes linéaires ou ramifiées en C₁₆-C₄₀, les hydroxyacides, les électrolytes, les épaississants, les esters d'acides gras, les esters d'acides gras et de glycérol, les tensioactifs, les parfums, les conservateurs, les filtres solaires, les protéines, les vitamines, les polymères, et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gel, de lait, de crème, de lotion plus ou moins épaissie ou de mousse.

Les compositions conformes à l'invention peuvent être utilisées pour le traitement des matières kératiniques telles que les cheveux, la peau, les cils, les sourcils, les ongles, les lèvres, le cuir chevelu et plus particulièrement les cheveux.

Les compositions peuvent également être utilisées pour le lavage et le nettoyage des matières kératiniques telles que les cheveux et la peau.

Les compositions selon l'invention sont utilisées généralement comme produits notamment pour le lavage, le soin, le conditionnement, le maintien de la coiffure ou la mise en forme des matières kératiniques telles que les cheveux.

Les compositions de l'invention peuvent plus particulièrement se présenter sous forme de shampooing, d'après-shampooing à rincer ou non, de compositions pour permanente, défrisage, coloration ou décoloration, ou encore sous forme de compositions à appliquer avant ou après une coloration, une décoloration, une permanente ou un défrisage ou encore entre les deux étapes d'une permanente ou d'un défrisage. De préférence, les compositions sont des compositions lavantes et moussantes pour les cheveux et/ou la peau.

En particulier, les compositions selon l'invention sont des compositions détergentes moussantes telles que des shampooings, des gels-douche et des bains moussants. Dans ce mode de réalisation de l'invention, les compositions comprennent une base tensioactive lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques.

La quantité minimale de base lavante est celle juste suffisante pour conférer à la composition finale un pouvoir moussant et/ou détergent satisfaisant.

Ainsi, selon l'invention, la base lavante peut représenter de 4 % à 30 % en poids, de préférence de 6 % à 25 % en poids, et encore plus préférentiellement de 8 % à 20 % en poids, du poids total de la composition finale.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou en mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkyl sulfates, les alkyl éther sulfates, alkyl amido éther sulfates, alkyl aryl polyéther sulfates, monoglycérides sulfates ; les alkyl sulfonates, alkylphosphates, alkyl amido sulfonates, alkyl aryl sulfonates, α-oléfine-sulfonates, paraffine-sulfonates ; les alkyl sulfo succinates, les alkyl éther sulfosuccinates, les alkyl amide sulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkylamido(C₆-C₂₄) éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras de polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s) ou zwittérionique(s) :

Les agents tensioactifs amphotères ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits commercialisés sous la dénomination MIRANOL, tels que décrits dans les brevets US-2528378 et US-2781354 et classés dans le dictionnaire CTFA, 3ème édition, 1982, sous les dénominations Amphocarboxy-glycinates et Amphocarboxypropionates de structures respectives

R₂-CONHCH₂CH₂-N(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R_{2'}-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂- CHOH - SO₃H
R_{2'} désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate commercialisé sous la dénomination commerciale MIRANOL C2M concentré par la Société RHODIA CHIMIE.

### (iv) Tensioactifs cationiques :

Parmi les tensioactifs cationiques, dont la nature ne revet pas dans le cadre de la présente invention de caractère critique, on peut citer en particulier (liste non limitative) : les sels d'amines grasses primaires, secondaires ou tertiaires, éventuellement polyoxy-alkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

L'invention a encore pour objet un procédé de traitement cosmétique des matières kératiniques telles que les cheveux consistant à appliquer sur ceux-ci une composition telle que définie précédemment puis à effectuer éventuellement un rinçage à l'eau, après un éventuel temps de pause.

Les compositions selon l'invention peuvent être préparées principalement selon deux mode opératoires :
Le premier consiste à chauffer à environ 80°C sous agitation tous les ingrédients de la composition puis à laisser refroidir le mélange jusqu'à température ambiante Le deuxième consiste à préparer un concentré nacrant qui comprenant des tensioactifs, l'agent opacifiant ou nacrant et l'alcool gras, de l'eau, un agent de pH et éventuellement un conservateur. L'agent opacifiant ou nacrant et l'alcool gras sont ajoutés sous agitation dans le mélange d'eau et de tensioactifs préalablement chauffés à environ 80°C. La température est maintenue pendant environ 30 minutes puis on refroidit le mélange jusqu'à 30°C environ. La quantité nécessaire de concentré est alors ajoutée dans la base shampooing à température ambiante à l'aide d'une turbine.

L'invention va être maintenant plus complètement illustrée à l'aide des exemples suivants qui ne sauraient être considérés comme la limitant aux modes de réalisation décrits. Dans ce qui suit, MA signifie Matière Active.

### EXEMPLE 1

On a préparé trois shampooings de compositions suivantes :
Les compositions A et B sont selon l'invention, la composition C est une composition comparative.

| - | A | B | C |
|---|---|---|---|
| - Lauryléther sulfate de sodium oxyéthyléné à 2,2 moles d'oxyde d'éthylène en solution aqueuse à 70% de MA | 14,5 gMA | 14,5 gMA | 14,5 gMA |
| - Cocoylbétaïne en solution aqueuse à 30% de MA | 2,3 gMA | 2,3 gMA | 2,3 gMA |
| - Diméthicone (MIRASIL DM 500000 de RHODIA CHIMIE) | ― | 2 g | ― |
| - Hydroxyéthylcellulose réticulée par l'épichlorhydrine et quaternisée par la triméthylamine (JR 400 de UNION CARBIDE) | ― | 0,3 gMA | ― |
| - Mélange d'alcool cétylique et de 1-(hexadécyloxy)-2-octadécanol(agent opacifiant) | 1,5 g | 1,5 g | 2,5 g |
| - Alcool gras en C22 (NAFOL 1822 C de CONDEA) | 1 g | 1 g | ― |
| - Monoéthanolamide d'acides de coprah | 0,95 g | 0,95 g | 0,95 g |
| - Conservateurs, parfum | qs | qs | qs |
| - Acide citrique, 1H2O qs | pH 5,5 | pH 5,5 | pH 5,5 |
| - Eau déminéralisée qsp | 100 g | 100 g | 100 g |

Le nacrage des compositions A et B selon l'invention est supérieur à celui de la composition C qui ne contient que l'agent opacifiant.
Les propriétés moussantes des compositions A et B sont bonnes.
Les compositions A et B ont une bonne viscosité et sont stables.

### EXEMPLE 2

On a préparé trois shampooings de compositions suivantes :
Les compositions A et B sont selon l'invention, la composition C est une composition comparative.

| - | A | B | C |
|---|---|---|---|
| - Lauryléther sulfate de sodium oxyéthyléné à 2,2 moles d'oxyde d'éthylène en solution aqueuse à 70% de MA | 14,5 gMA | 14,5 gMA | 14,5 gMA |
| - Cocoylbétaïne en solution aqueuse à 30% de MA | 2,3 gMA | 2,3 gMA | 2,3 gMA |
| - Diméthicone (MIRASIL DM 500000 de RHODIA CHIMIE) | ― | 2 g | ― |
| - Hydroxyéthylcellulose quaternisée par la triméthylamine (JR 400 de UNION CARBIDE) | ― | 0,3 gMA | ― |
| - Distéaryléther (agent opacifiant) | 1,5 g | 1,5 g | 2,5 g |
| - Alcool gras en C22 (NAFOL 1822 C de CONDEA) | 1 g | 1 g | ― |
| - Monoéthanolamide d'acides de coprah | 0,95 g | 0,95 g | 0,95 g |
| - Conservateurs, parfum | qs | qs | qs |
| - Acide citrique, 1H2O qs | pH 5,5 | pH 5,5 | pH 5,5 |
| - Eau déminéralisée qsp | 100 g | 100 g | 100 g |

Le nacrage des compositions A et B selon l'invention est supérieur à celui de la composition C qui ne contient que l'agent opacifiant.
Les propriétés moussantes des compositions A et B sont bonnes.
Les compositions A et B ont une bonne viscosité et sont stables.

Lorsqu'on remplace le NAFOL1822 C par un alcool gras comprenant environ 44% en poids d'alcool en C22 dans les compositions A ou B, on constate que le nacrage est tout à fait insuffisant.

## Revendications

1. Composition cosmétique caractérisée en qu'elle comprend dans un milieu cosmétiquement acceptable au moins une base tensioactive, au moins un alcool gras ou un mélange d'alcools gras linéaires et saturés à longue chaîne comportant au moins 50% en poids d'un alcool ayant 22 atomes de carbone et au moins un agent opacifiant et/ou nacrant.

2. Composition selon la revendication 1, **caractérisée en ce que** l'alcool gras ou le mélange d'alcools gras comporte au moins 70% en poids d'alcool ayant 22 atomes de carbone par rapport au poids total du mélange.

3. Composition selon l'une quelconque des revendications 1 ou 2, **caractérisée par le fait que** les agents nacrants et/ou opacifiant sont choisis parmi :
A) les dialkyléthers gras solides à une température inférieure ou égale à environ 30°C tels que par exemple les dialkyléthers de formule (I) :
R-O-R' (I)
dans laquelle :
R et R', identiques ou différents, désignent un radical alkyle, saturé ou insaturé, linéaire ou ramifié, comportant de 12 à 30 atomes de carbone et de préférence de 14 à 24 atomes de carbone, R et R' étant choisis de façon telle que le composé de formule (I) soit solide à une température inférieure ou égale à 30° C environ Plus particulièrement R et R' sont identiques.
B) les alcools ayant de 27 à 48 atomes de carbone et comportant un ou deux groupements éther et/ou thioéther ou sulfoxyde répondant à la formule (II):
R1-X-[C2H3(OH)]-CH2-Y-R2 (II)
dans laquelle R1 et R2 désignent indépendamment l'un de l'autre, des groupements alkyles linéaires en C12 à C24 ;
X désigne un atome d'oxygène, un atome de soufre, un groupement sulfoxyde ou méthylène ;
Y désigne un atome d'oxygène, un atome de soufre, un groupement sulfoxyde ou méthylène ;
dans le cas où Y désigne un groupement méthylène la somme du nombre d'atomes de carbone présents dans les groupements R1 et R2 a une valeur variant de 24 à 44 et de préférence de 28 à 40 inclus ;
lorsque Y ne désigne pas un groupement méthylène, la somme des atomes de carbone présents dans les groupements R1 et R2 a une valeur variant de 24 à 44 et de préférence de 28 à 40 inclus ;
lorsque X ou Y désigne sulfoxyde, Y ou X ne désigne pas soufre.

4. Composition selon l'une quelconque des revendications 1 à 3, **caractérisée en ce que** les agents nacrants et/ou opacifiant sont choisis parmi :
A) le distéaryléther,
B) les composés de formules (Il) pour lesquels X désigne oxygène, Y désigne méthylène, et R1 et R2 désignent des radicaux ayant 12 à 22 atomes de carbone.

5. Composition selon l'une quelconque des revendications 1 à 4, **caractérisée en ce que** l'alcool gras ou le mélange d'alcool gras représente de 0,5% à 15 % en poids, de préférence de 0,5 % à 5 % en poids, et encore plus préférentiellement de 0,5 % à 3 % en poids, du poids total de la composition finale.

6. Composition selon l'une quelconque des revendications 1 à 5, **caractérisée en ce que** l'agent opacifiant et/ou nacrant représente de 0,5% à 15 % en poids, de préférence de 0,5 % à 5 % en poids, et encore plus préférentiellement de 1 % à 3 % en poids, du poids total de la composition finale.

7. Composition selon l'une quelconque des revendications précédentes, **caractérisée en ce qu'**elle comprend en outre au moins un agent de conditionnement.

8. Composition selon la revendication précédente, **caractérisée en ce que** l'agent de conditionnement est choisi parmi les poly-α-oléfines, les huiles fluorées, les cires fluorées, les gommes fluorées, les esters d'acides carboxyliques, les polymères en particulier cationiques, les silicones, les huiles minérales, végétales ou animales, les céramides, les pseudocéramides, et leurs mélanges.

9. Composition selon l'une quelconque des revendications précédentes, **caractérisée par le fait qu'**elle se présente sous forme de gel, de lait, de crème, de lotion plus ou moins épaissie ou de mousse.

10. Composition selon l'une quelconque des revendications précédentes, caractérisée qu'elle constitue une composition détergente moussante telles que des shampooings, des gels-douche et des bains moussants.

11. Utilisation d'au moins un alcool gras ou un mélange d'alcools gras linéaires-et saturés à longue chaîne comportant au moins 50% en poids d'un alcool ayant 22 atomes de carbone et d'au moins un agent opacifiant et/ou nacrant comme agent de mise en suspension d'un agent de conditionnement insoluble dans une composition cosmétique en particulier de lavage et de conditionnement moussante contenant, dans un milieu aqueux cosmétiquement acceptable, une base tensioactive.

12. Utilisation d'un alcool gras ou un mélange d'alcools gras linéaires et saturés à longue chaîne comportant au moins 50% en poids d'un alcool ayant 22 atomes de carbone pour nacrer et/ou améliorer le nacrage des compositions comprenant au moins une base tensioactive et au moins un agent opacifiant et/ou nacrant.

13. Utilisation d'un alcool gras ou d'un mélange d'alcools gras linéaires et saturés à longue chaîne comportant au moins 50% en poids d'un alcool ayant 22 atomes de carbone et d'un agent opacifiant et/ou nacrant en tant que base nacrante.

14. Procédé de traitement cosmétique des matières kératiniques en particulier des cheveux, **caractérisé par le fait qu'**il consiste à appliquer sur lesdites matières une composition telle que définie selon l'une quelconque des revendications 1 à 20 puis à effectuer éventuellement un rinçage à l'eau.

## Patentansprüche

1. Kosmetische Zusammensetzung, **dadurch gekennzeichnet, daß** sie in einem kosmetisch akzeptablen Medium mindestens eine grenzflächenaktive Basisformulierung, mindestens einen geradkettigen und gesättigten langkettigen Fettalkohol oder ein Gemisch geradkettiger und gesättigter langkettiger Fettalkohole, der/das mindestens 50 Gew.-% eines Alkohols umfaßt, der 22 Kohlenstoffatome aufweist, und mindestens ein Trübungsmittel und/oder Perlglanzmittel enthält.

2. Zusammensetzung nach Anspruch 1, **dadurch gekennzeichnet, daß** der Fettalkohol oder das Gemisch von Fettalkoholen mindestens 70 Gew.-% des Alkohols umfaßt, der 22 Kohlenstoffatome aufweist, bezogen auf das Gesamtgewicht des Gemischs.

3. Zusammensetzung nach einem der Ansprüche 1 und 2, **dadurch gekennzeichnet, daß** die Perlglanzmittel und/oder Trübungsmittel ausgewählt sind unter:
A) den Dialkylfettethern, die bei einer Temperatur von etwa 30 °C oder darunter fest sind, wie z.B. den Dialkylethern der Formel (I)
R-O-R' (I),
in der:
R und R', gleich oder verschieden, eine gesättigte oder ungesättigte, geradkettige oder verzweigte Alkylgruppe, die 12 bis 30 Kohlenstoffatome und vorzugsweise 14 bis 24 Kohlenstoffatome aufweist, bedeuten, wobei R und R' so ausgewählt sind, daß die Verbindung der Formel (I) bei einer Temperatur von etwa 30 °C oder darunter fest ist,
R und R' insbesondere gleiche Gruppen darstellen,
B) den Alkoholen, die 27 bis 48 Kohlenstoffatome aufweisen und ein oder zwei Ether- und/oder Thioether- oder Sulfoxidgruppen enthalten, die der allgemeinen Formel (II)
R1-X-[C₂H₃(OH)]-CH₂-Y-R2 (II)
entsprechen, in der
R1 und R2 unabhängig voneinander geradkettige C₁₂-C₂₄-Alkylgruppen bedeuten;
X ein Sauerstoffatom, ein Schwefelatom, eine Sulfoxidgruppe oder eine Methylengruppe bedeutet;
Y ein Sauerstoffatom, ein Schwefelatom, eine Sulfoxidgruppe oder eine Methylengruppe bedeutet;
die Summe der Zahl der Kohlenstoffatome, die in den Gruppen R1 und R2 enthalten sind, einen Wert im Bereich von 24 bis 44 und vorzugsweise 28 bis 40 einschließlich der Grenzwerte aufweist, wenn Y eine Methylengruppe bedeutet;
die Summe der Kohlenstoffatome, die in den Gruppen R1 und R2 enthalten sind, einen Wert im Bereich von 24 bis 44 und vorzugsweise 28 bis 40 einschließlich der Grenzwerte aufweist, wenn Y keine Methylengruppe bedeutet;
Y oder X nicht Schwefel bedeutet, wenn X oder Y eine Sulfoxidgruppe bedeutet.

4. Zusammensetzung nach einem der Ansprüche 1 bis 3, **dadurch gekennzeichnet, daß** die Perlglanzmittel und/oder Trübungmittel ausgewählt sind unter
A) dem Distearylether,
B) den Verbindungen der Formel (II), für die X Sauerstoff bedeutet, Y Methylen bedeutet und R1 und R2 Gruppen bedeuten, die 12 bis 22 Kohlenstoffatome aufweisen.

5. Zusammensetzung nach einem der Ansprüche 1 bis 4, **dadurch gekennzeichnet, daß** der Fettalkohol oder das Fettalkoholgemisch 0,5 bis 15 Gew.-%, vorzugsweise 0,5 bis 5 Gew.-% und noch bevorzugter 0,5 bis 3 Gew.-% des Gesamtgewichts der fertigen Zusammensetzung ausmacht.

6. Zusammensetzung nach einem der Ansprüche 1 bis 5, **dadurch gekennzeichnet, daß** das Trübungsmittel und/oder Perlglanzmittel 0,5 bis 15 Gew.-%, vorzugsweise 0,5 bis 5 Gew.-% und noch bevorzugter 1 bis 3 Gew.-% des Gesamtgewichts der fertigen Zusammensetzung ausmacht.

7. Zusammensetzung nach einem der vorhergehenden Ansprüche, **dadurch gekennzeichnet, daß** sie außerdem mindestens ein Konditioniermittel enthält.

8. Zusammensetzung nach dem vorhergehenden Anspruch, **dadurch gekennzeichnet, daß** das Konditioniermittel unter den Poly-α-olefinen, den fluorierten Ölen, den fluorierten Wachsen, den fluorierten Gummis, den Carbonsäureestern, den Polymeren, insbesondere kationischen Polymeren, den Siliconen, den Mineralölen, pflanzlichen oder tierischen Ölen, den Ceramiden, den Pseudoceramiden und ihren Gemischen ausgewählt ist.

9. Zusammensetzung nach einem der vorhergehenden Ansprüche, **dadurch gekennzeichnet, daß** sie in Form eines Gels, einer Milch, einer Creme, einer mehr oder weniger verdickten Lotion oder eines Schaums vorliegt.

10. Zusammensetzung nach einem der vorhergehenden Ansprüche, **dadurch gekennzeichnet, daß** sie eine schäumende reinigende Zusammensetzung bildet, wie z.B. ein Haarwaschmittel, ein Duschgel und/oder ein Schaumbad.

11. Verwendung mindestens eines geradkettigen und gesättigten langkettigen Fettalkohols oder eines Gemischs geradkettiger und gesättigter langkettiger Fettalkohole, der/das mindestens 50 Gew.-% eines Alkohols umfaßt, der 22 Kohlenstoffatome aufweist, und mindestens eines Trübungsmittels und/oder Perlglanzmittels als Mittel zum Suspendieren eines Konditioniermittels, das in einer kosmetischen Zusammensetzung, insbesondere einer schäumenden kosmetischen Zusammensetzung zum Waschen und Konditionieren, unlöslich ist, die in einem kosmetisch akzeptablen Medium eine grenzflächenaktive Basisformulierung enthält.

12. Verwendung eines geradkettigen und gesättigten langkettigen Fettalkohols oder eines Gemischs geradkettiger und gesättigter langkettiger Fettalkohole, der/das mindestens 50 Gew.-% eines Alkohols umfaßt, der 22 Kohlenstoffatome aufweist, um Zusammensetzungen Perlglanz zu verleihen oder deren Perlglanz zu verbessern, die mindestens eine grenzflächenaktive Basisformulierung und mindestens ein Trübungsmittel und/oder Perlglanzmittel enthalten.

13. Verwendung eines geradkettigen und gesättigten langkettigen Fettalkohols oder eines Gemischs derartiger Fettalkohole, der/das mindestens 50 Gew.-% eines Alkohols enthält, der 22 Kohlenstoffatome aufweist, und eines Trübungsmittels und/oder Perlglanzmittels als Perlglanz-Basisformulierung.

14. Verfahren zur kosmetischen Behandlung von Keratinmaterialien, insbesondere der Haare, **dadurch gekennzeichnet, daß** es darin besteht, auf die Keratinmaterialien eine wie in einem der Ansprüche 1 bis 20 definierte Zusammensetzung aufzutragen und dann gegebenenfalls mit Wasser auszuspülen.

## Claims

1. Cosmetic composition, **characterized in that** it comprises, in a cosmetically acceptable medium, at least one surfactant base, at least one linear, saturated, long-chain fatty alcohol or mixture of fatty alcohols comprising at least 50% by weight of an alcohol containing 22 carbon atoms, and at least one opacifier and/or pearlescent agent.

2. Composition according to Claim 1, **characterized in that** the fatty alcohol or mixture of fatty alcohols comprises at least 70% by weight of alcohol containing 22 carbon atoms relative to the total weight of the mixture.

3. Composition according to either of Claims 1 and 2, **characterized in that** the pearlescent agents and/or opacifier are chosen from:
A) fatty dialkyl ethers which are solid at a temperature of less than or equal to about 30°C, such as, for example, the dialkyl ethers of formula (I):
R-O-R' (I)
in which:
R and R', which may be identical or different, denote a saturated or unsaturated, linear or branched alkyl radical comprising from 12 to 30 carbon atoms and preferably from 14 to 24 carbon atoms, R and R' being chosen such that the compound of formula (I) is solid at a temperature of less than or equal to about 30°C. More particularly, R and R' are identical.
B) alcohols containing from 27 to 48 carbon atoms and comprising one or two ether and/or thioether or sulphoxide groups corresponding to formula (II):
R1-X-[C2H3(OH)]-CH2-Y-R2 (II)
in which R1 and R2 denote, independently of each other, linear C12 to C24 alkyl groups;
X denotes an oxygen atom, a sulphur atom or a sulphoxide or methylene group;
Y denotes an oxygen atom, a sulphur atom or a sulphoxide or methylene group;
when Y denotes a methylene group, the sum of the number of carbon atoms present in the groups R1 and R2 has a value ranging from 24 to 44 and preferably from 28 to 40 inclusive;
when Y does not denote a methylene group, the sum of the carbon atoms present in the groups R1 and R2 has a value ranging from 24 to 44 and preferably from 28 to 40 inclusive;
when X or Y denotes sulphoxide, Y or X does not denote sulphur.

4. Composition according to any one of Claims 1 to 3, **characterized in that** the pearlescent agents and/or opacifier are chosen from:
A) distearyl ether,
B) compounds of formula (II) for which X denotes oxygen, Y denotes methylene and R1 and R2 denote radicals containing 12 to 22 carbon atoms.

5. Composition according to any one of Claims 1 to 4, **characterized in that** the fatty alcohol or mixture of fatty alcohols represents from 0.5% to 15% by weight, preferably from 0.5% to 5% by weight and even more preferably from 0.5% to 3% by weight, relative to the total weight of the final composition.

6. Composition according to any one of Claims 1 to 5, **characterized in that** the opacifier and/or pearlescent agent represents from 0.5% to 15% by weight, preferably from 0.5% to 5% by weight and even more preferably from 1% to 3% by weight, relative to the total weight of the final composition.

7. Composition according to any one of the preceding claims, **characterized in that** it also comprises at least one conditioner.

8. Composition according to the preceding claim, **characterized in that** the conditioner is chosen from poly-α-olefins, fluoro oils, fluoro waxes, fluoro gums, carboxylic acid esters, polymers, particularly cationic polymers, silicones, mineral, plant or animal oils, ceramides and pseudoceramides, and mixtures thereof.

9. Composition according to any one of the preceding claims, **characterized in that** it is in the form of a gel, a milk, a cream, a more or less thickened lotion or a foam.

10. Composition according to any one of the preceding claims, **characterized in that** it is a foaming detergent composition, such as shampoos, shower gels or bubble baths.

11. Use of at least one linear, saturated, long-chain fatty alcohol or mixture of fatty alcohols comprising at least 50% by weight of an alcohol containing 22 carbon atoms, and at least one opacifier and/or pearlescent agent, as an agent for suspending an insoluble conditioner in a cosmetic composition, in particular a foaming conditioning and washing composition, containing a surfactant base in a cosmetically acceptable aqueous medium.

12. Use of a linear, saturated, long-chain fatty alcohol or mixture of fatty alcohols comprising at least 50% by weight of an alcohol containing 22 carbon atoms, to give a pearling effect to and/or to enhance the pearling effect of compositions comprising at least one surfactant base and at least one opacifier and/or pearlescent agent.

13. Use of a linear, saturated, long-chain fatty alcohol or mixture of fatty alcohols comprising at least 50% by weight of an alcohol containing 22 carbon atoms, and of an opacifier and/or pearlescent agent, as a pearling base.

14. Process for the cosmetic treatment of keratin substances, in particular the hair, **characterized in that** it consists in applying a composition as defined according to any one of Claims 1 to 20 to the said substances and then optionally rinsing them with water.
